# EUROPÄISCHE PATENTSCHRIFT

(11) **EP 0 895 997 B1**
(45) Veröffentlichungstag und Bekanntmachung des Hinweises auf die Patenterteilung: **30.10.2002**
(21) Anmeldenummer: 98114885.1
(22) Anmeldetag: 07.08.1998
(51) Int. Cl.: C07F 9/54, C07C 11/21

(54) **Verfahren zur Herstellung von Phosphoniumsalzen**
Process for the preparation of phosphonium salts
Procédé pour la préparation de sels de phosphonium

(30) Priorität: 08.08.1997 DE 19734446
(43) Veröffentlichungstag der Anmeldung: 10.02.1999
(73) Patentinhaber: BASF AKTIENGESELLSCHAFT, 67056 Ludwigshafen (DE)
(72) Erfinder: Wegner, Christoph, 67105 Schifferstadt (DE); Paust, Joachim, 67141 Neuhofen (DE); John, Michael, 67245 Lambsheim (DE)

(56) Entgegenhaltungen:
- EP-A- 0 382 067
- DE-B- 1 155 126
- DE-B- 1 203 264

## Beschreibung

Die vorliegende Erfindung betrifft ein Verfahren zur Herstellung von 3,7,11-Trimethyldodeca-2,4,6,10-tetraen-1-yl-phosphoniumsalzen, die entsprechenden Phosphoniumsalze sowie ein Verfahren zur Herstellung von Lycopin.

In der Regel werden die C₁₅-Phosphoniumsalze zur Synthese von Carotinoiden aus Vinylcarbinolen durch Reaktion mit Triphenylphosphan und einer starken Säure wie HCl oder H₂SO₄ in protischen Lösungsmitteln hergestellt (siehe z.B. J. Chem. Soc., 1965, 2019 - 2026). Anders als Vinyl-β-Ionol, ein für die Synthese von Vitamin A und β-Carotin eingesetztes Edukt, läßt sich Vinylpseudoionol als Edukt zur Herstellung von Lycopin unter diesen Standardkonditionen nur mit schlechten Ausbeuten und mit niedrigen E/Z-Selektivitäten zu den entsprechenden C₁₅-Phosphoniumsalzen umsetzen.

In der EP 382 067 ist ein Verfahren beschrieben, nach dem C₁₅-Phosphoniumsalze niederer Alkansäuren als Zwischenprodukte hergestellt werden, da die genannten Salze starker Säuren grundsätzlich schlechte E/Z-Selektivitäten und bei der folgenden Herstellung von Lycopin geringe Ausbeuten (Nebenprodukte) ergeben. Die Salze der Alkansäuren müssen vor der abschließenden Wittig-Olefinierung in einer aufwendigen Prozedur mittels Anionenaustausch wieder in die Chloride überführt werden. Zur Erzielung eines hohen E/Z-Verhältnisses im Lycopin ist zusätzlich eine Abtrennung von (Z)-Anteilen des Phosphoniumsalzes durch Kristallisation erforderlich.

Die DE-AS 27 29 974 offenbart ein Verfahren zur Herstellung wässriger Lösungen von Polyenyltriarylphosphoniumsalzen von starken Säuren in Essigsäure (siehe Beispiel 3), allerdings werden in den Beispielen keine 3,7,11-Trimethyldodeca-2,4,6,10-tetraen-1-yl-phosphoniumsalze und auch keine Alkansulfonsäure genannt.

Es bestand die Aufgabe, ein Verfahren zur Herstellung von Phosphoniumsalzen zur Verfügung zu stellen, bei dem in einem Verfahrensschritt 3,7,11-Trimethyldodeca-2,4,6,10-tetraen-1-ylphosphoniumsalze mit hohem E-Anteil erhalten werden, die bei der Weiterreaktion zu Lycopin hohe E/Z-Selektivitäten ergeben.

Die Aufgabe wird mit einem Verfahren zur Herstellung von Phosphoniumsalzen der allgemeinen Formel I 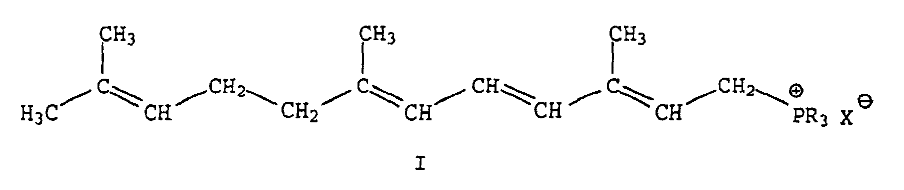 gelöst,
wobei
- R =: Aryl und
- X^{⊖} =: CₙH₂ₙ₊₁-SO₃^{⊖} mit n = 1 - 4,
Ar-SO₃^{⊖} mit Ar = Phenyl, Toluyl oder
CF₃-SO₃^{⊖} bedeuten, das
dadurch gekennzeichnet ist, daß man einen Alkohol der Formel II 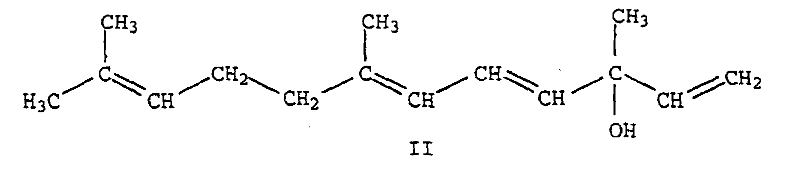 mit Triarylphosphan und Sulfonsäuren der allgemeinen Formeln

CₙH₂ₙ₊₁-SO₃H, Ar-SO₃H oder CF₃-SO₃H,

worin n und Ar die oben angegebenen Bedeutungen haben,
in einem Lösungsmittel umsetzt.

Dabei wird in der Regel die Sulfonsäure im Lösungsmittel vorgelegt und der Alkohol zugegeben.

Als Sulfonsäuren werden bevorzugt Alkansulfonsäuren, z.B. Ethanoder Methansulfonsäure eingesetzt, insbesonders die technisch verfügbare Methansulfonsäure (70 %). Die Umsetzung wird bevorzugt in einer Alkancarbonsäure als Lösungsmittel vorgenommen, wobei Alkancarbonsäuren mit 1 - 8 C-Atomen besonders geeignet sind, insbesondere Essigsäure und Propionsäure.

Die Erfindung betrifft auch Phosphoniumsalze der allgemeinen Formel I 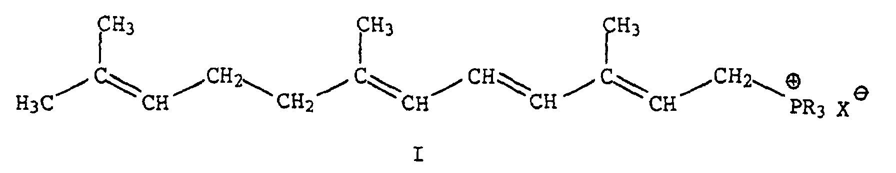 worin R = Aryl und X^{⊖} = CₙH₂ₙ₊₁-SO₃^{⊖} mit n = 1 - 4, oder CF₃-SO₃^{⊖} insbesondere CH₃-SO₃^{⊖}, bedeuten. Der Begriff "Aryl" bezeichnet übliche, in Phosphinen vorkommende Arylreste, wie Phenyl, Toluyl, Naphthyl, ggf. jeweils substituiert, insbesondere Phenyl.

Die Erfindung betrifft weiterhin ein Verfahren zur Herstellung von Lycopin, dadurch gekennzeichnet, daß man ein Phosphoniumsalz der allgemeinen Formel I mit den in Anspruch 1 abgegebenen Bedeutungen für R, X, Ar und n mit 2,7-Dimethyl-2,4,6-octatriendial umsetzt.

Mit dem erfindungsgemäßen Verfahren läßt sich Vinylpseudoionol der Formel II mit z.B. Methansulfonsäure in Eisessig in Ausbeuten von 85 - 90 % und mit einer E/Z-Selektivität von 3,7 : 1 zum entsprechenden Phosphoniummethansulfonat umsetzen. Das so erhaltene Salz eignet sich direkt für die Wittig-Reaktion mit 2,7-Dimethyl-2,4,6-octatriendial zur Herstellung von Lycopin, wobei auf zusätzliche Schritte wie Anionenaustausch oder Anreicherung der E-Komponente durch eine zusätzliche Kristallisation verzichtet werden kann.

Es war überraschend, daß mit starken Säuren wie den Sulfonsäuren diese vorteilhaften Ergebnisse erzielbar sind, denn in der EP 382 067 A1 wird festgestellt, die Herstellung von Lycopin aus Salzen starker Säuren - Halogenide, Sulfate oder Phosphate sind genannt - ergebe nur geringe Ausbeuten, wobei insbesondere bei der Herstellung der Phosphoniumsalze unreaktive Nebenprodukte gebildet werden (s. S. 2, Z. 5 - 9). Beim erfindungsemäßen Verfahren wird die Sulfonsäure in der Regel vorgelegt, bevor Vinylpseudoionol (Alkohol der Formel II) zugetropft wird, wobei eine hohe E/Z-Selektivität erreicht wird.

Das erfindungsgemäße Verfahren wird insbesondere bei Temperaturen von 20 bis 120°C, bevorzugt bei 60 bis 100°C, in an sich bekannter Weise ausgeführt.

Der Alkohol der allgemeinen Formel II (3,7,11-Trimethyldodeca-1,4,6,10-tetraen-3-ol) wird auch als Vinyl-Ψ-ionol oder Vinylpseudoionol bezeichnet und ist z.B. aus J. Chem. Soc. 1965, 2023 oder der EP 382 067 bekannt. Er kann nach bekannten Methoden aus Pseudoionon erhalten werden.

Das Phosphoniumsalz der allgemeinen Formel I liegt bevorzugt in der all-E-Form vor.

Das mit dem erfindungsgemäßen Verfahren hergestellte Lycopin wird in üblicher Weise als Farbmittel für Lebensmittel oder Futtermittel, als Antioxidans oder als Nutraceutical eingesetzt.

### Beispiel 1

Ein Gemisch von 41,9 g Triphenylphosphan, 13,7 g Methansulfonsäure (70 %ig) und 100 ml Essigsäure wurde auf 80°C erwärmt und dann innerhalb von 10 Minuten mit 24,2 g 3,7,11-Trimethyldodeca-1,4E,6E,10-tetraen-3-ol (91 %ig) versetzt. Das Gemisch wurde noch 30 Minuten gerührt, bevor man die Essigsäure bei 50 mbar abdestillierte. Der Rückstand, bestehend aus 3,7,11-Trimethyldodeca-2,4,6,10-tetraen-1-yl-triphenylphosphoniummethansulfonat, wurde zur Entfernung letzter Essigsäurereste in 100 ml Toluol aufgenommen, welches anschließend ebenfalls bei 50 mbar abdestilliert wurde. Dieser Vorgang wurde dann noch einmal wiederholt. Der Rückstand wurde in 25 ml Methanol aufgenommen, einmal mit 100 ml und viermal mit je 50 ml Heptan extrahiert. Die methanolische Lösung enthaltend das gewünschte Produkt wurde mittels HPLC und internem Standard untersucht. Die Analyse ergab 50,7 g (90,4 %) 3,7,11-Trimethyldodeca-2,4,6,10-tetraen-1-yl-triphenylphosphoniummethansulfonat bestehend aus 39,8 g (71,0 %) all E-Isomer, 5,69 g (10,1 %) 6Z-Isomer und 5,22 g (9,3 %) 2Z-Isomer. Das Verhältnis des E-Isomers zu den Z-Isomeren beträgt demnach 3,7 : 1.

### Beispiel 2

Ein Gemisch von 39,3 g Triphenylphosphan, 15,1 g Trifluormethansulfonsäure und 100 ml Essigsäure wurde auf 80°C erwärmt und dann innerhalb von 10 Minuten mit 23,2 g (95 %ig) 3,7,11-Trimethyldodeca-1,4E,6E,10-tetraen-3-ol versetzt. Das Gemisch wurde noch 30 Minuten gerührt, bevor man die Reaktionslösung mittels HPLC und internem Standard auf Gehalt und Selektivität analysierte. Die Lösung enthielt 56,7 g (92,2 %) 3,7,11-Trimethyldodeca-2,4,6,10-tetraen-1-yl-triphenylphosphoniumtrifluormethansulfonat bestehend aus 43,7 g (71,1 %) all-E-Isomer, 6,33 g (10,3 %) 6Z-Isomer und 6,64 g (10,8 %) 2Z-Isomer. Das Verhältnis E-Isomer/Z-Isomeren beträgt demnach 3,4 : 1.

### Beispiel 3

Ein Gemisch von 39,3 g Triphenylphosphan, 19,0 g p-Toluolsulfonsäure und 100 ml Essigsäure wurde auf 80°C erwärmt und dann innerhalb von 10 Minuten mit 23,2 g (95 %ig) 3,7,11-Trimethyldodeca-1,4E,6E,10-tetraen-3-ol versetzt. Das Gemisch wurde noch 30 Minuten gerührt, bevor man die Reaktionslösung mittels HPLC und internem Standard auf Gehalt und Selektivität analysierte. Die Lösung enthielt 53,5 g (84,0 %) 3,7,11-Trimethyldodeca-2,4,6,10-tetraen-1-yl-triphenylphosphonium-p-toluolsulfonat bestehend aus 41,5 g (65,2 %) all-E-Isomer, 6,39 g (10,0 %) 6Z-Isomer und 5,59 g (8,8 %) 2Z-Isomer. Das Verhältnis E-Isomer/Z-Isomeren beträgt demnach 3,5 : 1.

### Beispiel 4:

Ein Gemisch von 41,9 g Triphenylphosphan, 11,0 g Ethansulfonsäure und 100 ml Essigsäure wurde auf 80°C erwärmt und dann innerhalb von 10 Minuten mit 24,5 g (90 %ig) 3,7,11-Trimethyldodeca-1,4E,6E,10-tetraen-3-ol versetzt. Das Gemisch wurde noch 30 Minuten gerührt, bevor man die Reaktionslösung mittels HPLC und internem Standard auf Gehalt und Selektivität analysierte. Die Lösung enthielt 49,2 g (85,6 %) 3,7,11-Trimethyldodeca-2,4,6,10-tetraen-1-yl-triphenylphosphoniumethansulfonat bestehend aus 38,8 g (67,5 %) all-E-Isomer, 5,62 g (9,8 %) 6Z-Isomer und 4,76 g (8,3 %) 2Z-Isomer. Das Verhältnis E-Isomer/Z-Isomeren beträgt demnach 3,7 : 1.

### Beispiel 5

Ein Gemisch von 41,9 g Triphenylphosphan, 13,9 g Methansulfonsäure (69,2 %ig) und 100 ml Propionsäure wurde auf 80°C erwärmt und dann innerhalb von 10 Minuten mit 24,5 g (90 %ig) 3,7,11-Trimethyldodeca-1,4E,6E,10-tetraen-3-ol versetzt. Das Gemisch wurde noch 30 Minuten gerührt, bevor man die Reaktionslösung mittels HPLC und internem Standard auf Gehalt und Selektivität analysierte. Die Lösung enthielt 49,9 g (89,0 %) 3,7,11-Trimethyldodeca-2,4,6,10-tetraen-1-yl-triphenylphosphoniummethansulfonat bestehend aus 39,5 g (70,4 %) all-E-Isomer, 5,81 g (10,3 %) 6Z-Isomer und 4,60 g (8,2 %) 2Z-Isomer. Das Verhältnis E-Isomer/Z-Isomeren beträgt demnach 3,8 : 1.

### Vergleichsbeispiel

Das Beispiel 1 der EP 372 067 wurde mehrmals, wie in der Druckschrift angegeben, nachgearbeitet. Vor der Anreicherung des E-Isomers erhält man in 70 bis 80 % Ausbeute das C15-Phosphoniumsalz mit einem E/Z-Verhältnis von 2,4-2,6:1.

## Patentansprüche

1. Verfahren zur Herstellung von Phosphoniumsalzen der allgemeinen Formel I 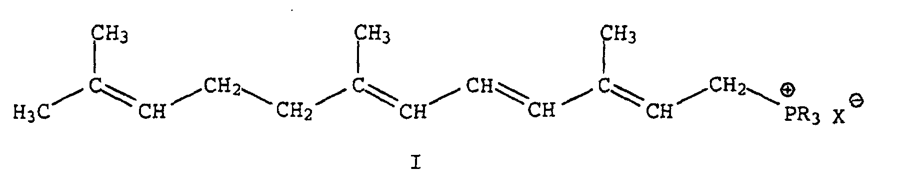 wobei
R = Aryl und
X^{⊖} = CₙH₂ₙ₊₁-SO₃^{⊖} mit n = 1 - 4,
Ar-SO₃^{⊖} mit Ar = Phenyl, Toluyl oder
CF₃-SO₃^{⊖} bedeuten,
**dadurch gekennzeichnet, daß** man einen Alkohol der allgemeinen Formel II 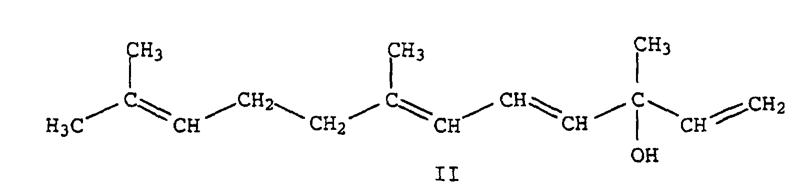 mit Triarylphosphan und Sulfonsäuren der Formeln
CₙH₂ₙ₊₁-SO₃H, Ar-SO₃H oder CF₃-SO₃H,
worin n und Ar die oben angegebenen Bedeutungen haben,
in einem Lösungsmittel umsetzt.

2. Verfahren nach Anspruch 1, **dadurch gekennzeichnet, daß** die Sulfonsäure eine Alkansulfonsäure mit n = 1 - 2 ist.

3. Verfahren nach Anspruch 1, **dadurch gekennzeichnet, daß** die Umsetzung in einer Alkancarbonsäure als Lösungsmittel, bevorzugt in Essigsäure oder Propionsäure erfolgt.

4. Verfahren nach Anspruch 1, **dadurch gekennzeichnet, daß** R Phenyl bedeutet.

5. Phosphoniumsalz der allgemeinen Formel I 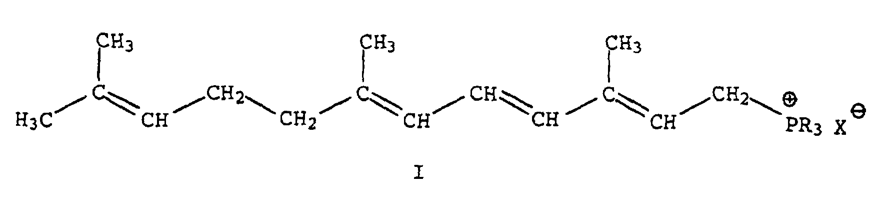 wobei
R = Aryl und
X^{⊖} = CₙH₂ₙ₊₁-SO₃^{⊖} mit n = 1 - 4 oder CF₃-SO₃^{⊖} bedeuten.

6. Phosphoniumsalz nach Anspruch 5, **dadurch gekennzeichnet, daß** R Phenyl und CₙH₂ₙ₊₁ Methyl bedeutet.

7. Verfahren zur Herstellung von Lycopin, **dadurch gekennzeichnet, daß** man ein Phosphoniumsalz der allgemeinen Formel I mit den in Anspruch 1 abgegebenen Bedeutungen für R, X, Ar und n mit 2,7-Dimethyl-2,4,6-octatriendial umsetzt.

## Claims

1. A process for preparing phosphonium salts of the formula I 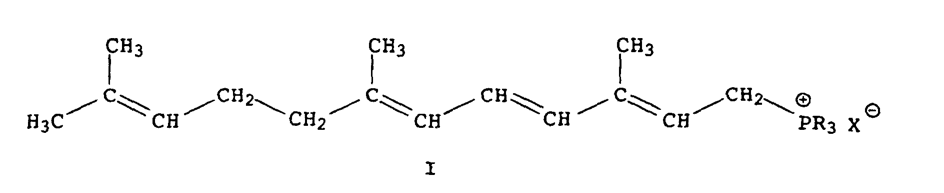 where
R is aryl and
X^{⊖} is CₙH₂ₙ₊₁-SO₃^{⊖} with n = 1 - 4,
Ar-SO₃^{⊖} with Ar = phenyl, tolyl or
CF₃-SO₃^{⊖},
which comprises reacting an alcohol of the formula II 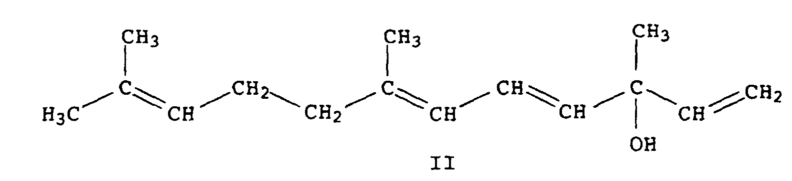 with triarylphosphine and sulfonic acids of the formulae
CₙH₂ₙ₊₁-SO₃H, Ar-SO₃H or CF₃-SO₃H,
where n and Ar have the abovementioned meanings,
in a solvent.

2. A process as claimed in claim 1, wherein the sulfonic acid is an alkanesulfonic acid with n = 1 - 2.

3. A process as claimed in claim 1, wherein the reaction takes place in an alkanecarboxylic acid as solvent, preferably in acetic acid or propionic acid.

4. A process as claimed in claim 1, wherein R is phenyl.

5. A phosphonium salt of the formula I 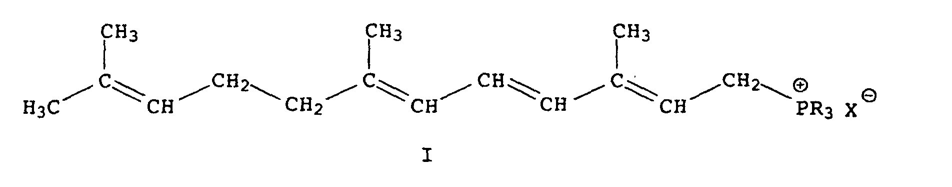 where
R is aryl and
X^{⊖} is CₙH₂ₙ₊₁-SO₃^{⊖} with n = 1 - 4 or CF₃-SO₃^{⊖}.

6. A phosphonium salt as claimed in claim 5, wherein R is phenyl and CₙH₂ₙ₊₁ is methyl.

7. A process for preparing lycopene, which comprises reacting a phosphonium salt of the formula I having the meanings for R, X, Ar and n stated in claim 1 with 2,7-dimethyl-2,4,6-octatrienedial.

## Revendications

1. Procédé pour la préparation de sels de phosphonium de formule générale I 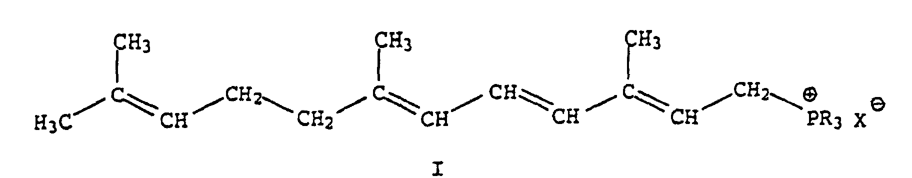
R = aryle et
X⁻ = CₙH₂ₙ₊₁-SO₃^{⊖} avec n = 1 - 4,
Ar-SO₃^{⊖} avec Ar = phényle, toluyle ou
CF₃-SO₃^{⊖},
**caractérisé par le fait qu'**on fait réagir dans un solvant un alcool de formule générale II 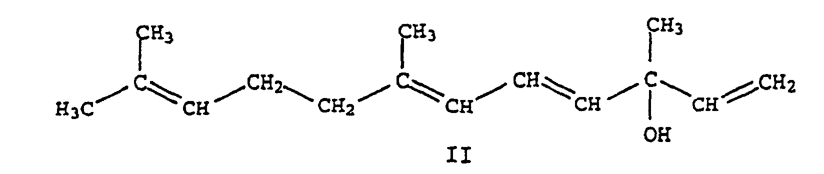 avec du triarylphosphane et des acides sulfoniques de formules
CₙH₂ₙ₊₁-SO₃H, Ar-SO₃H ou CF₃-SO₃H,
où n et Ar ont les significations indiquées plus haut.

2. Procédé selon la revendication 1, **caractérisé par le fait que** l'acide sulfonique est un acide alcane-sulfonique avec n = 1 - 2.

3. Procédé selon la revendication 1, **caractérisé par le fait que** la réaction s'effectue dans un acide alcanecarboxylique comme solvant, de préférence dans l'acide acétique ou l'acide propionique.

4. Procédé selon la revendication 1, **caractérisé par le fait que** R représente le phényle.

5. Sel de phosphonium de formule générale I 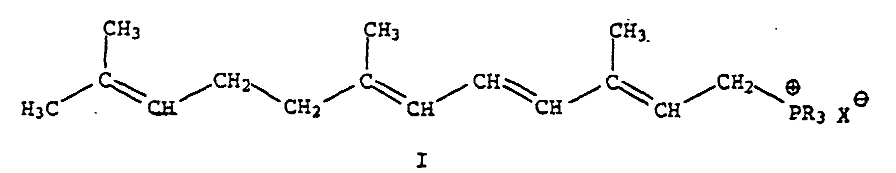 où
R = aryle et
X⁻ = CₙH₂ₙ₊₁-SO₃^{⊖} avec n = 1 - 4 ou CF₃-SO₃^{⊖}.

6. Sel de phosphonium selon la revendication 5, **caractérisé par le fait que** R désigne le phényle et CₙH₂ₙ₊₁ le méthyle.

7. Procédé pour la préparation de lycopène, **caractérisé par le fait qu'**on fait réagir un sel de phosphonium de formule générale I ayant les significations indiquées dans la revendication 1 pour R, X, Ar et n avec le 2,7-diméthyl-2,4,6-octatriènedial.
